(19) Europäisches Patentamt
European Patent Office
Office européen des brevets

(11) EP 3 153 158 A1

(12) **EUROPÄISCHE PATENTANMELDUNG**

(43) Veröffentlichungstag:
**12.04.2017 Patentblatt 2017/15**

(51) Int Cl.:
***A61K 9/20** (2006.01)* ***A61K 31/426** (2006.01)*

(21) Anmeldenummer: **15002887.6**

(22) Anmeldetag: **09.10.2015**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA**

(71) Anmelder: **STADA ARZNEIMITTEL AG**
**61118 Bad Vilbel (DE)**

(72) Erfinder:
- **SCHÖNBORN, Jessica**
  **61118 Bad Vilbel (DE)**
- **WINTER, Sven**
  **61381 Friedrichsdorf (DE)**

(74) Vertreter: **Kernebeck, Thomas**
**Kernebeck Patentanwalts GmbH**
**Stiftstraße 2**
**60313 Frankfurt am Main (DE)**

(54) **ORALE FEBUXOSTAT-TABLETTE**

(57) Die vorliegende Erfindung betrifft eine feste pharmazeutische orale Darreichungsform in Form einer Tablette, umfassend Febuxostat als Wirkstoff sowie ein Trockenbindemittel. Um eine derartige Darreichungsform bereitzustellen, die durch verhältnismäßig niedrige Werte der Friabilität ausgezeichnet und entsprechend gut befilmbar ist, wird vorgeschlagen, dass das Trockenbindemittel einen D10-Wert von 1 $\mu$m bis 15 $\mu$m, einen D50-Wert von 10 $\mu$m bis 50 $\mu$m und einen D90-Wert von 30 $\mu$m bis 120 $\mu$m aufweist.

EP 3 153 158 A1

**Beschreibung**

Die vorliegende Erfindung betrifft eine feste pharmazeutische orale Darreichungsform in Form einer Tablette, umfassend Febuxostat als Wirkstoff sowie ein Trockenbindemittel.

Febuxostat ist der internationale Freiname (INN (International Nonproprietary Name)) für 2-(3-Cyan-4-isobutyloxyphenyl)-4-methylthiazol-5-carbonsäure. Febuxostat weist die folgende Strukturformel auf:

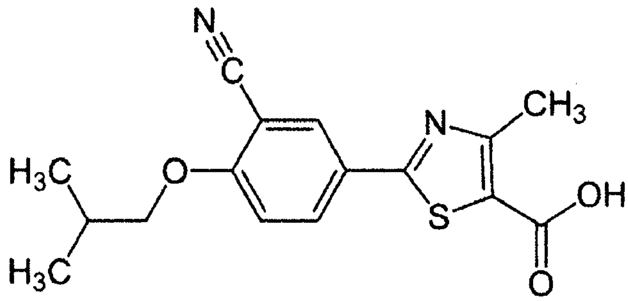

Febuxostat ist ein Arzneistoff aus der Gruppe der Urikostatika und ist zur Behandlung der chronischen Hyperurikämie bei Erkrankungen zugelassen, die bereits zu Ablagerungen von Uratkristallen geführt haben. Der Wirkungsmechanismus von Febuxostat beruht auf der selektiven Hemmung der Xanthinoxidase, die Hypoxanthin zunächst in Xanthin und dann weiter in Harnsäure umwandelt. Die durch die Hemmung bewirkte Erhöhung der Hypoxanthin-Konzentration bewirkt zudem eine Hemmung der Purinsynthese.

Um eine angemessene pharmazeutische Wirkung zu gewährleisten, muss Febuxostat in verhältnismäßig hohen Dosen verabreicht werden. Zur Vermeidung einer gleichzeitigen Einnahme mehrerer Febuxostat-Darreichungsformen und einer damit einhergehenden erhöhten Patientenakzeptanz sind im Stand der Technik Filmtabletten bekannt - wie z.B. Filmtabletten der Handelsbezeichnung Adenuric® - die einen relativ hohen Wirkstoffgehalt aufweisen. Diese Filmtabletten erlauben die Verabreichung der zu applizierenden Dosis durch Einnahme einer einzigen Darreichungsform.

Aufgrund des verhältnismäßig hohen Wirkstoffanteils und der Tatsache, dass das Febuxostat wegen seiner schlechten Wasserlöslichkeit in mikronisierter Form eingesetzt wird, besitzt eine entsprechende Pulvermischung für die trockene Fertigung eine nur unzureichende Fließfähigkeit, weshalb das Originator-Produkt Adenuric® mittels Feuchtgranulierung hergestellt wird.

Febuxostat kann aber in einer Vielzahl von kristallinen polymorphen Formen vorliegen sowie auch in amorpher Form, weshalb eine Feuchtgranulierung nachteilig ist, da diese in der Regel die Umwandlung einer gegebenen polymorphen Form in eine andere begünstigt. Zur Behebung dieses Nachteils schlägt WO 2013/001441 eine feste orale Febuxostat-Formulierung vor, die mittels eines trockenen Prozesses hergestellt ist, beispielsweise mittels Direktverpressung oder mittels Trockengranulierung. Febuxostat besitzt jedoch relativ schlechte Verpressungseigenschaften, weshalb mittels trockenen Verfahren hergestellte Tabletten im Allgemeinen verhältnismäßig schlechte mechanische Eigenschaften aufweisen. Insbesondere weisen entsprechende Tabletten hohe Friabilitäts-Werte auf, weshalb die Tabletten beispielsweise nur schwer befilmbar sind bzw. die aufgetragenen Filme nicht ausreichend auf der Oberfläche der Tablette anhaften und sich entsprechend leicht ablösen.

Aufgabe der vorliegenden Erfindung ist es daher, eine feste pharmazeutische orale Darreichungsform in Form einer Tablette bereitzustellen, die Febuxostat als Wirkstoff sowie ein Trockenbindemittel umfasst, wobei sich die Darreichungsform durch verhältnismäßig niedrige Werte der Friabilität auszeichnet und entsprechend gut befilmbar ist.

Diese Aufgabe wird erfindungsgemäß gelöst durch eine feste pharmazeutische orale Darreichungsform in Form einer Tablette, umfassend Febuxostat als Wirkstoff sowie ein Trockenbindemittel, wobei das Trockenbindemittel einen D10-Wert von 1 $\mu$m bis 15 $\mu$m, einen D50-Wert von 10 $\mu$m bis 50 $\mu$m und einen D90-Wert von 30 $\mu$m bis 120 $\mu$m aufweist.

Überraschenderweise wurde festgestellt, dass die erfindungsgemäße Darreichungsform vergleichsweise geringe Werte der Friabilität aufweist. So kann die erfindungsgemäße Darreichungsform Friabilitäts-Werte von bis zu kleiner/gleich 0,1 % aufweisen, wobei die erreichbaren Werte auch abhängig sind vom Anteil des Trockenbindemittels in der Darreichungsform sowie vom Verpressungsdruck. Die Friabilität ist gemäß dem Europäischen Arzneibuch (Ph.Eur.), 8. Ausgabe, S. 411-412, Kapitel 2.9.7 "Friabilität von nicht überzogenen Tabletten" zu bestimmen.

Ferner weist die erfindungsgemäße Darreichungsform eine verhältnismäßig hohe Bruchfestigkeit F sowie eine relative hohe Druckfestigkeit $\sigma$ auf.

Die Bruchfestigkeit F ist erfindungsgemäß nach Ph.Eur., 8. Auflage, S. 412, Kapitel 2.9.8 "Bruchfestigkeit von Tabletten" zu bestimmen. Dabei wird erfindungsgemäß bevorzugt die Bruchfestigkeit F mittels eines Tablettenhärte-Testers durch uniaxiale vertikale Belastung zwischen zwei horizontalen Backen mit planparallelen Flächen gemessen

durch Aufnahme einer kontrollierten Kraft-Weg-Kurve, wobei bei der Messung die Probe auf der unteren Backe platziert und worauf die obere Backe progressiv herabgefahren wird. Erfindungsgemäß bevorzugt wird hierzu ein Tabletten-Härtetester der Firma ERWEKA International AG, Schweiz, Gerätetyp: TBH 250 IC, eingesetzt. Dabei werden runde Tabletten beispielsweise, d.h. Tabletten mit einem kreisförmigen Querschnitt wie z.B. runde flache biplane Tabletten, bei der Bruchfestigkeitsmessung über den Steg (d.h. radial) zerbrochen.

Die Bruchfestigkeit F einer Tablette kann von ihrer Form und ihren Abmessungen abhängen. Es kann daher von Vorteil sein, die mechanische Festigkeit einer Tablette als Druckfestigkeit σ anzugeben, da die Druckfestigkeit σ die Geometrie der Tablette berücksichtigt und somit eine davon im Prinzip unabhängige Größe darstellt. Die Druckfestigkeit σ wird mittels der experimentell bestimmten Bruchfestigkeit F berechnet. Für zylinderförmige Tabletten beispielsweise wird die Druckfestigkeit σ mittels der nachstehenden mathematischen Beziehung berechnet (siehe "Die Tablette", A. Ritschel et al., 2. Auflage, Editio-Cantor-Verlag, 2002, ISBN 3-87193-228-0):

$$\sigma = \frac{2F}{\pi D t}$$

in welcher

- σ die Druckfestigkeit ist;
- F die Bruchfestigkeit ist;
- n die Zahl pi ist;
- D der Tablettendurchmesser ist;
- t die Tablettendicke ist.

Für gewölbte Tabletten beispielsweise wird die Druckfestigkeit σ mittels der nachstehenden mathematischen Beziehung berechnet (siehe "Die Tablette", A. Ritschel et al., 2. Auflage, Editio-Cantor-Verlag, 2002, ISBN 3-87193-228-0):

$$\sigma = \frac{10F}{\pi D^2}\left(2{,}84\frac{t}{D} - 0{,}126\frac{t}{W} + 3{,}15\frac{W}{D} + 0{,}01\right)^{-1}$$

in welcher

- σ die Druckfestigkeit ist;
- F die Bruchfestigkeit ist;
- n die Zahl pi ist;
- D der Tablettendurchmesser ist;
- t die Dicke über alles;
- W die Dicke des zentralen Zylinders ist.

In der erfindungsgemäßen Darreichungsform ist ein Trockenbindemittel enthalten. Dieses weist in freier pulvriger Form einen D10-Wert von 1 $\mu$m bis 15 $\mu$m, einen D50-Wert von 10 $\mu$m bis 50 $\mu$m und einen D90-Wert von 30 $\mu$m bis 120 $\mu$m auf. Unter D10-Wert wird in diesem Zusammenhang die Partikelgröße verstanden, bei der 10 % der Partikel bezogen auf das Volumen kleiner als der D10-Wert sind und 90 % der Partikel bezogen auf das Volumen größer sind als der D10-Wert. In Analogie dazu wird unter D50-Wert die Partikelgröße verstanden, bei der 50 % der Partikel bezogen auf das Volumen kleiner als der D50-Wert sind und 50 % der Partikel bezogen auf das Volumen größer sind als der D50-Wert. In Analogie dazu wird unter D90-Wert die Partikelgröße verstanden, bei der 90 % der Partikel bezogen auf das Volumen kleiner als der D90-Wert sind und 10 % der Partikel bezogen auf das Volumen größer sind als der D90-Wert. Der D10-, der D50- und der D90-Wert des Trockenbindemittels ist gemäß Ph. Eur., 8. Ausgabe, Kapitel 2.9.31 "Bestimmung der Partikelgröße durch Laserdiffraktometrie", Seiten 455-460 zu bestimmen. Dabei wird erfindungsgemäß bevorzugt der D10-, der D50- und der D90-Wert mittels Laserbeugung unter Einsatz eines Gerätes mit der Bezeichnung "Mastersizer 2000" der Firma "Malvern Instruments" und dem Datenanalyseprogramm Malvern Application v5.60 ermittelt. Die Geräte-/Programmeinstellungen sind dabei wie folgt: Dispergiersystem (dispersing system) Scirocco 2000; Messbereich (result range) 0,02 bis 2000 $\mu$m; Auswertung (reporting) Mie; Berechnungsmodel (calculation model) Mie; Berechnungsempfindlichkeit (calculation sensitivity) universell (general purpose); Messzeit (measurement time) 5 s; Hintergrundzeit (background time) 5 s; Messaufnahmen (measurement snaps) 5000; Hintergrundaufnahmen (background snaps) 5000; Dispergiermethode (dispersing method) Trockenmessung, das Pulver wird in Luft dispergiert;

Einsatz (tray) universell (general purpose); Probennehmereinstellungen (sampler settings) Vibrationszuführrate (vibration feed rate) 60 %, Luftdruck zur Dispergierung (dispersive air pressure) 2,5 bar; Trübungsgrenzen (obscuration limits) Untergrenze 2, Obergrenze 10; Anzahl der Messungen 2.

Entsprechend einer bevorzugten Ausführungsform der erfindungsgemäßen Darreichungsform ist es vorgesehen, dass das Trockenbindemittel einen D10-Wert von 1 $\mu$m bis 5 $\mu$m, einen D50-Wert von 10 $\mu$m bis 15 $\mu$m und einen D90-Wert von 30 $\mu$m bis 40 $\mu$m aufweist. Es konnte festgestellt werden, dass eine erfindungsgemäße Darreichungsform mit einem Trockenbindemittel mit einem D10-, D50- und D90-Wert in den genannten Bereichen einen besonders niedrigen Abrieb (Friabilität) aufweist.

In der erfindungsgemäßen Darreichungsform ist ein Trockenbindemittel mit einer bestimmten Partikelgrößenverteilung enthalten. Die Natur des Trockenbindemittels kann dabei im Prinzip beliebig sein, solange es der geforderten Partikelgrößenverteilung genügt. Erfindungsgemäß bevorzugt ist es jedoch, dass das Trockenbindemittel ausgewählt ist der Gruppe bestehend aus Cellulose und Cellulose-Derivate, wie mikrokristalline Cellulose, Hydroxypropylcellulose, Hydroxypropylmethylcellulose, Methylcellulose und Ethylcellulose, Lactose, modifizierte Stärke, Calciumphosphat, Magnesium-Aluminium-Silikat, Mono-und Polysaccharide, wie Dextran, Dextrin, Maltodextrin, Dextrat, Galactomannan, Glucose und direktkomprimierbare Zucker auf der Basis von Saccharose, Alginsäure und Alginate, wie Natriumalginat und Propylenglykolalginat, Zuckeralkohole, wie Sorbit, Mannit und Palatinit, Gelatine, Soja-Protein, Zein, Polyvinylpyrrolidon, Copovidon (Vinylpyrrolidon-Vinylacetat-Copolymer), Polymethacrylat, Polyacrylsäure, Polyacrylamid, Natriumstärkeglykolat, Polyethylenglykol 4000 bis 6000, Milchzucker, Rohrzucker und Calciumcarbonat.

Gemäß einer weiter bevorzugten Ausführungsform der erfindungsgemäßen Darreichungsform ist es vorgesehen, dass das Trockenbindemittel ein Vinylpyrrolidon-Vinylacetat-Copolymer ist. Vinylpyrrolidon-Vinylacetat-Copolymer-Trockenbindemittel können in der erfindungsgemäßen Darreichungsform in verhältnismäßig geringer Menge enthalten sein und bewirken dennoch eine hohe Abriebfestigkeit derselben.

Ein erfindungsgemäß besonders bevorzugtes Vinylpyrrolidon-Vinylacetat-Copolymer-Trockenbindemittel wird von der BASF AG, Ludwigshafen, Deutschland unter der Bezeichnung Kollidon® VA 64 (fein) angeboten. Dieses Trockenbindemittel weist ein Vinylpyrrolidon/Vinylacetat-Massenverhältnis von 6:4 auf.

Gemäß einer weiter bevorzugten Ausführungsform der erfindungsgemäßen Darreichungsform ist es vorgesehen, dass der Anteil der Darreichungsform an dem Trockenbindemittel 0,05 Gew.-% bis 20 Gew.-% beträgt, vorzugsweise 0,5 Gew.-% bis 10 Gew.-%. Der Anteil der erfindungsgemäßen Darreichungsform an Trockenbindemittel kann vergleichsweise gering gewählt sein, ohne dadurch die Friabilität der Darreichungsform signifikant zu erhöhen. Dadurch wird ein maximaler Wirkstoffgehalt in der Darreichungsform gewährleistet.

Nach einer weiter bevorzugten Ausführungsform der erfindungsgemäßen Darreichungsform beträgt der Anteil der Darreichungsform an Febuxostat 1 Gew.-% bis 40 Gew.-%, vorzugsweise 10 Gew.-% bis 30 Gew.-%. Die erfindungsgemäße Darreichungsform kann Febuxostat in den genannten, relativ hohen Anteilen enthalten und weist dennoch eine verhältnismäßig niedrige Friabilität auf.

Es konnte festgestellt werden, dass die erfindungsgemäße Darreichungsform auch dann verhältnismäßig niedrige Friabilitäts-Werte aufweist, wenn der Wirkstoff Febuxostat in mikronisierter Form in der Darreichungsform enthalten ist. Entsprechend ist es gemäß einer weiter bevorzugten Ausführungsform der erfindungsgemäßen Darreichungsform vorgesehen, dass das Febuxostat (in freier pulvriger Form) einen D10-Wert von 1 $\mu$m bis 5 $\mu$m, einen D50-Wert von 1 $\mu$m bis 15 $\mu$m und einen D90-Wert von 1,5 $\mu$m bis 20 $\mu$m aufweist. Unter D10-Wert wird auch in diesem Zusammenhang die Partikelgröße verstanden, bei der 10 % der Partikel bezogen auf das Volumen kleiner als der D10-Wert sind und 90 % der Partikel bezogen auf das Volumen größer sind als der D10-Wert. In Analogie dazu wird unter D50-Wert die Partikelgröße verstanden, bei der 50 % der Partikel bezogen auf das Volumen kleiner als der D50-Wert sind und 50 % der Partikel bezogen auf das Volumen größer sind als der D50-Wert. In Analogie dazu wird unter D90-Wert die Partikelgröße verstanden, bei der 90 % der Partikel bezogen auf das Volumen kleiner als der D90-Wert sind und 10 % der Partikel bezogen auf das Volumen größer sind als der D90-Wert.

Der D10-, der D50- und der D90-Wert des Febuxostats ist gemäß Ph. Eur., 8. Ausgabe, Kapitel 2.9.31 "Bestimmung der Partikelgröße durch Laserdiffraktometrie", Seiten 455-460 zu bestimmen. Dabei wird erfindungsgemäß bevorzugt der D10-, der D50- und der D90-Wert mittels Laserbeugung unter Einsatz eines Gerätes mit der Bezeichnung "Mastersizer 2000" der Firma "Malvern Instruments" und dem Datenanalyseprogramm Malvern Application v5.60 ermittelt. Die Geräte-/Programmeinstellungen sind dabei gleich zu den vorstehend bezüglich des Trockenbindemittels genannten.

Gemäß einer weiter bevorzugten Ausführungsform der erfindungsgemäßen Darreichungsform ist es vorgesehen, dass die Darreichungsform mittels eines trockenen Verfahrens, das heißt unter Ausschluss von Lösungsmitteln hergestellt ist. Dabei ist es erfindungsgemäß besonders bevorzugt, wenn die Darreichungsform mittels Direkttablettierung oder mittels Trockengranulierung hergestellt ist. Direkttablettierungs- und Trockengranulierungsverfahren sind im Stand der Technik bekannt, z.B. aus "Die Tablette", A. Bauer-Brandl et al., 3. Auflage, Editio-Cantor-Verlag, 2012, ISBN 978-3-87193-407-0.

Entsprechend einer weiter bevorzugten Ausführungsform der erfindungsgemäßen Darreichungsform ist es

vorgesehen, dass das Febuxostat in der kristallinen polymorphen Form A, M oder G vorliegt.

Die kristalline polymorphe Form A ist in EP 1 020 454 offenbart und weist ein unter Verwendung von Cu-Kalpha-Strahlung (lambda = 1,5418 Angström) erhaltenes Pulver-Röntgenbeugungsmuster mit Signalen bei Beugungswinkeln 2thetha von 6,62°± 0,2°, 7,18° ± 0,2°, 12,80° ± 0,2°, 13,26° ± 0,2°, 16,48° ± 0,2°, 19,58° ± 0,2°, 21,92° ± 0,2°, 22,68° ± 0,2°, 25,84° ± 0,2°, 26,70° ± 0,2°, 29,16° ± 0,2° und 36,70° ± 0,2° auf.

Die kristalline polymorphe Form M ist in CN 101085761 offenbart und weist ein unter Verwendung von Cu-Kalpha-Strahlung (lambda = 1,5418 Angström) erhaltenes Pulver-Röntgenbeugungsmuster mit Signalen bei Beugungswinkeln 2thetha von 5,8°± 0,2°, 7,9° ± 0,2°, 11,6° ± 0,2°, 12,7° ± 0,2°, 20,5° ± 0,2° und 25,9° ± 0,2° auf.

Die kristalline polymorphe Form G ist in EP 1 020 454 offenbart und weist ein unter Verwendung von Cu-Kalpha-Strahlung (lambda = 1,5418 Angström) erhaltenes Pulver-Röntgenbeugungsmuster mit Signalen bei Beugungswinkeln 2thetha von 6,86°± 0,2°, 8,36° ± 0,2°, 9,60° ± 0,2°, 11,76° ± 0,2°, 13,74° ± 0,2°, 14,60° ± 0,2°, 15,94° ± 0,2°, 16,74° ± 0,2°, 17,56° ± 0,2°, 20,00° ± 0,2°, 21,26° ± 0,2°, 23,72° ± 0,2°, 24,78° ± 0,2°, 25,14° ± 0,2°, 25,74° ± 0,2°, 26,06° ± 0,2°, 26,64° ± 0,2°, 27,92° ± 0,2°, 28,60° ± 0,2°, 29,66° ± 0,2° und 29,98° ± 0,2° auf.

Gemäß einer weiter bevorzugten Ausführungsform der erfindungsgemäßen Darreichungsform ist es vorgesehen, dass die Darreichungsform eine innere granuläre Phase sowie eine äußere kontinuierliche Phase aufweist, wobei das Febuxostat in der inneren Phase und das Trockenbindemittel in der äußeren Phase enthalten ist. Es wurde festgestellt, dass sich die erfindungsgemäße Darreichungsform auch dann durch eine geringe Friabilität auszeichnet, wenn das Febuxostat in einer inneren granulären Phase und das Trockenbindemittel in einer äußeren kontinuierlichen Phase enthalten ist.

Nach einer weiter bevorzugten Ausführungsform der erfindungsgemäßen Darreichungsform ist es vorgesehen, dass das die innere Phase bildende Granulat (in freier pulvriger Form) einen D10-Wert von 1 $\mu$m bis 60 $\mu$m, einen D50-Wert von 20 $\mu$m bis 800 $\mu$m und einen D90-Wert von 100 $\mu$m bis 1600 $\mu$m aufweist. Unter D10-Wert wird auch in diesem Zusammenhang die Partikelgröße verstanden, bei der 10 % der Partikel bezogen auf das Volumen kleiner als der D10-Wert sind und 90 % der Partikel bezogen auf das Volumen größer sind als der D10-Wert. In Analogie dazu wird unter D50-Wert die Partikelgröße verstanden, bei der 50 % der Partikel bezogen auf das Volumen kleiner als der D50-Wert sind und 50 % der Partikel bezogen auf das Volumen größer sind als der D50-Wert. In Analogie dazu wird unter D90-Wert die Partikelgröße verstanden, bei der 90 % der Partikel bezogen auf das Volumen kleiner als der D90-Wert sind und 10 % der Partikel bezogen auf das Volumen größer sind als der D90-Wert.

Der D10-, der D50- und der D90-Wert der inneren granulären Phase ist gemäß Ph. Eur., 8. Ausgabe, Kapitel 2.9.31 "Bestimmung der Partikelgröße durch Laserdiffraktometrie", Seiten 455-460 zu bestimmen. Dabei wird erfindungsgemäß bevorzugt der D10-, der D50- und der D90-Wert mittels Laserbeugung unter Einsatz eines Gerätes mit der Bezeichnung "Mastersizer 2000" der Firma "Malvern Instruments" und dem Datenanalyseprogramm Malvern Application v5.60 ermittelt. Die Geräte-/Programmeinstellungen sind dabei gleich zu den vorstehend bezüglich des Trockenbindemittels genannten.

Entsprechend einer weiter bevorzugten Ausführungsform der erfindungsgemäßen Darreichungsform ist es vorgesehen, dass die Darreichungsform eine Friabilität von kleiner als 0,5 % aufweist, bevorzugt eine Friabilität von kleiner als 0,1 % und mehr bevorzugt eine Friabilität von kleiner als 0,05 %. In einer weiter bevorzugten Ausführungsform der erfindungsgemäßen Darreichungsform ist es vorgesehen, dass die Darreichungsform eine Friabilität zwischen 0,05 % und 0,01 % aufweist.

Gemäß einer weiter bevorzugten Ausführungsform der erfindungsgemäßen Darreichungsform ist es vorgesehen, dass die Darreichungsform frei von Laktose ist. Es konnte festgestellt werden, dass bei Laktose-freien erfindungsgemäßen Darreichungsformen weniger Bruch auftritt, beispielsweise während der Friabilitäts-Bestimmung.

Entsprechend einer weiter bevorzugten Ausführungsform der erfindungsgemäßen Darreichungsform ist es vorgesehen, dass die Darreichungsform ferner einen oder mehrere pharmazeutische Hilfsstoffe umfasst. Erfindungsgemäß bevorzugte Hilfsstoffe sind ausgewählt aus der Gruppe bestehend aus Fließregulierungsmittel, Sprengmittel, Füllmittel und Schmiermittel.

Der Anteil der erfindungsgemäßen Darreichungsform an pharmazeutischem/n Hilfsstoff/en beträgt erfindungsgemäß bevorzugt 20 Gew.-% bis 90 Gew.-% und weiter bevorzugt 50 Gew.-% bis 80 Gew.-%.

Die erfindungsgemäße Darreichungsform weist erfindungsgemäß bevorzugt eine Befilmung auf.

Die vorliegende Erfindung betrifft ferner eine pulverförmige Mischung zur Herstellung der erfindungsgemäßen Darreichungsform, wobei die Mischung Febuxostat sowie ein Trockenbindemittel umfasst und wobei das Trockenbindemittel einen D10-Wert von 1 $\mu$m bis 15 $\mu$m, einen D50-Wert von 10 $\mu$m bis 50 $\mu$m und einen D90-Wert von 30 $\mu$m bis 120 $\mu$m aufweist.

Darüber hinaus betrifft die vorliegende Erfindung die Verwendung eines Trockenbindemittels mit einem D10-Wert von 1 $\mu$m bis 15 $\mu$m, einem D50-Wert von 10 $\mu$m bis 50 $\mu$m und einem D90-Wert von 30 $\mu$m bis 120 $\mu$m bei der Herstellung der erfindungsgemäßen Darreichungsform.

Die vorliegende Erfindung betrifft ferner ein Verfahren, insbesondere zur Herstellung der erfindungsgemäßen Darreichungsform, umfassend die Schritte

- Bereitstellen einer pulverförmigen Mischung, enthaltend Febuxostat;
- Trockengranulieren der Mischung unter Erhalt eines Trockengranulates;
- Vermischen des Trockengranulats mit einem Trockenbindemittel und optional einem oder mehreren pharmazeutischen Hilfsstoffen, wobei das Trockenbindemittel einen D10-Wert von 1 $\mu$m bis 15 $\mu$m, einen D50-Wert von 10 $\mu$m bis 50 $\mu$m und einen D90-Wert von 30 $\mu$m bis 120 $\mu$m aufweist;
- Verpressen der Mischung von Trockengranulat und Trockenbindemittel unter Erhalt einer Tablette und optional
- Befilmen der Tablette.

Die nachfolgenden Ausführungsbeispiele dienen der Erläuterung der Erfindung.

**Ausführungsbeispiel 1:**

Es wurden Tabletten mit einer Zusammensetzung gemäß Tabelle 1 hergestellt.

Tabelle 1:

| **Substanz** | **Menge pro Tablette [mg]** | **Funktion der Substanz** |
|---|---|---|
| **innere Phase (Trockengranulat)** | | |
| Febuxostat (Form G) | 80,00 | Wirkstoff |
| hochdisperses Siliciumdioxid (Aerosil®) | 1,50 | Fließregulierungsmittel |
| Natriumcarboxymethylstärke (Primojel®) | 12,00 | Sprengmittel |
| mikrokristalline Cellulose (Avicel® PH 101) | 66,00 | Füllmittel / Bindemittel |
| Magnesiumstearat | 1,50 | Schmiermittel |
| **äußere Phase** | | |
| Copovidon (Kollidon® VA 64 fine; D10=4 $\mu$m, D50=13 $\mu$m, D90=33 $\mu$m) | 12,00 | Trockenbindemittel |
| mikrokristalline Cellulose (Avicel® PH 102) | 202,50 | Füllmittel |
| hochdisperses Siliciumdioxid (Aerosil®) | 2,50 | Fließregulierungsmittel |
| Natriumcarboxymethylstärke (Primojel®) | 16,00 | Sprengmittel |
| Magnesiumstearat | 6,00 | Schmiermittel |
| **Film** | | |
| Opadry® II yellow (enthält Polyvinylalkohol, Titandioxid, Polyethylenglycol, Talkum und gelbes Eisenoxid) | 12,00 | vollständiges Befilmungssystem |
| **Gesamtgewicht der Filmtablette** | 412,000 | |

Die Tabletten wurden wie folgt hergestellt.

Zunächst wurde die innere granuläre Phase hergestellt. Dazu wurden Febuxostat, mikrokristalline Cellulose, Natriumcarboxymethylstärke, hochdisperses Siliciumdioxid und Magnesiumstearat gesiebt und miteinander vermischt. Die resultierende pulverförmige Wirkstoffmischung wurde dann mittels eines Walzenkompaktors granuliert und anschließend gesiebt, wobei das resultierende Trockengranulat einen Hausner-Faktor von 1,0 bis 1,3 aufwies.

Natriumcarboxymethylstärke, Copovidon, mikrokristalline Cellulose, hochdisperses Siliciumdioxid und Magnesiumstearat wurden mit dem Granulat unter Erhalt der finalen, zu tablettierenden Mischung gemischt.

Die so erhaltene zu tablettierende Mischung wurde mittels einer konventionellen Tablettenpresse zu oblongen, bikonvexen Tabletten mit einseitiger Bruchkerbe und einer Länge von 16 mm und einer Breite von 5 mm verpresst. Die so erhaltenen unbefilmten Tabletten wiesen eine Friabilität von 0,03 % auf, wobei nach dem Friabilitäts-Test kein Tablettenbruch beobachtet werden konnte. Die unbefilmten Tabletten wiesen ferner eine Bruchfestigkeit F von 140 N und eine Druckfestigkeit $\sigma$ von 0,46 N/mm$^2$ auf, wobei bei der Bestimmung der Bruchfestigkeit die Backen des Messgerätes auf die kurzen Stirnseiten der Tablette gefahren wurden.

Die wie oben beschrieben erhaltenen Tabletten wurden in einer konventionellen Filmbeschichtungsvorrichtung durch Besprühen mit einer Beschichtungssuspension befilmt. Die Beschichtungssuspension wurde hergestellt durch

Suspendieren des kommerziell erhältlichen Befilmungssystems Opadry® II yellow in Wasser. Der Film haftete auf den Tablettenkernen sehr gut.

**Ausführungsbeispiel 2:**

Es wurden Tabletten mit einer Zusammensetzung gemäß Tabelle 2 hergestellt.

Tabelle 2:

| **Substanz** | **Menge pro Tablette [mg]** | **Funktion der Substanz** |
|---|---|---|
| **innere Phase (Trockengranulat)** | | |
| Febuxostat (Form M) | 120,00 | Wirkstoff |
| hochdisperses Siliciumdioxid (Aerosil®) | 2,25 | Fließregulierungsmittel |
| Natriumcarboxymethylstärke (Primojel®) | 18,00 | Sprengmittel |
| mikrokristalline Cellulose (Avicel® PH 101) | 99,00 | Füllmittel / Bindemittel |
| Magnesiumstearat | 2,25 | Schmiermittel |
| **äußere Phase** | | |
| Copovidon (Kollidon® VA 64 fine; D10=4 $\mu$m, D50=13 $\mu$m, D90=33 $\mu$m) | 18,00 | Trockenbindemittel |
| mikrokristalline Cellulose (Avicel® PH 102) | 303,75 | Füllmittel |
| hochdisperses Siliciumdioxid (Aerosil®) | 3,75 | Fließregulierungsmittel |
| Natriumcarboxymethylstärke (Primojel®) | 24,00 | Sprengmittel |
| Magnesiumstearat | 9,00 | Schmiermittel |
| **Film** | | |
| Opadry® II yellow (enthält Polyvinylalkohol, Titandioxid, Polyethylenglycol, Talkum und gelbes Eisenoxid) | 18,00 | vollständiges Befilmungssystem |
| **Gesamtgewicht der Filmtablette** | 618,000 | |

Die Tabletten wurden analog Ausführungsbeispiel 1 hergestellt. Die unbefilmten oblongen bikonvexen Tabletten mit einer Länge von 17,5 mm und einer Breite von 7,8 mm wiesen eine Friabilität von 0,03 % auf, wobei nach dem Friabilitäts-Test kein Tablettenbruch beobachtet werden konnte. Die unbefilmten Tabletten wiesen ferner eine Bruchfestigkeit F von 180 N und eine Druckfestigkeit $\sigma$ von 0,47 N/mm$^2$ auf, wobei bei der Bestimmung der Bruchfestigkeit die Backen des Messgerätes auf die kurzen Stirnseiten der Tablette gefahren wurden.

**Ausführungsbeispiel 3:**

Es wurden Tabletten mit einer Zusammensetzung gemäß Tabelle 3 hergestellt.

Tabelle 3:

| **Substanz** | **Menge pro Tablette [mg]** | **Funktion der Substanz** |
|---|---|---|
| **innere Phase (Trockengranulat)** | | |
| Febuxostat (Form A) | 120,00 | Wirkstoff |
| hochdisperses Siliciumdioxid (Aerosil®) | 2,25 | Fließregulierungsmittel |
| Natriumcarboxymethylstärke (Primojel®) | 18,00 | Sprengmittel |
| mikrokristalline Cellulose (Avicel® PH 101) | 99,00 | Füllmittel / Bindemittel |
| Magnesiumstearat | 2,25 | Schmiermittel |

(fortgesetzt)

| | | |
|---|---|---|
| **äußere Phase** | | |
| Copovidon (Kollidon® VA 64 fine; D10=4 $\mu$m, D50=13 $\mu$m, D90=33 $\mu$m) | 18,00 | Trockenbindemittel |
| mikrokristalline Cellulose (Avicel® PH 102) | 303,75 | Füllmittel |
| hochdisperses Siliciumdioxid (Aerosil®) | 3,75 | Fließregulierungsmittel |
| Natriumcarboxymethylstärke (Primojel®) | 24,00 | Sprengmittel |
| Magnesiumstearat | 9,00 | Schmiermittel |
| **Film** | | |
| Opadry® II yellow (enthält Polyvinylalkohol, Titandioxid, Polyethylenglycol, Talkum und gelbes Eisenoxid) | 18,00 | vollständiges Befilmungssystem |
| **Gesamtgewicht der Filmtablette** | 618,000 | |

Die Tabletten wurden analog Ausführungsbeispiel 1 hergestellt. Die unbefilmten oblongen bikonvexen Tabletten mit einer Länge von 17,5 mm und einer Breite von 7,8 mm wiesen eine Friabilität von 0,02 % auf, wobei nach dem Friabilitäts-Test kein Tablettenbruch beobachtet werden konnte. Die unbefilmten Tabletten wiesen ferner eine Bruchfestigkeit F von 160 N und eine Druckfestigkeit $\sigma$ von 0,47 $N/mm^2$ auf, wobei bei der Bestimmung der Bruchfestigkeit die Backen des Messgerätes auf die kurzen Stirnseiten der Tablette gefahren wurden.

## Patentansprüche

1. Feste pharmazeutische orale Darreichungsform in Form einer Tablette, umfassend Febuxostat als Wirkstoff sowie ein Trockenbindemittel, **dadurch gekennzeichnet, dass** das Trockenbindemittel einen D10-Wert von 1 $\mu$m bis 15 $\mu$m, einen D50-Wert von 10 $\mu$m bis 50 $\mu$m und einen D90-Wert von 30 $\mu$m bis 120 $\mu$m aufweist.

2. Darreichungsform nach Anspruch 1, **dadurch gekennzeichnet, dass** das Trockenbindemittel einen D10-Wert von 1 $\mu$m bis 5 $\mu$m, einen D50-Wert von 10 $\mu$m bis 15 $\mu$m und einen D90-Wert von 30 $\mu$m bis 40 $\mu$m aufweist.

3. Darreichungsform nach einem der voranstehenden Ansprüche, **dadurch gekennzeichnet, dass** das Trockenbindemittel ausgewählt ist der Gruppe bestehend aus Cellulose und Cellulose-Derivate, wie mikrokristalline Cellulose, Hydroxypropylcellulose, Hydroxypropylmethylcellulose, Methylcellulose und Ethylcellulose, Lactose, modifizierte Stärke, Calciumphosphat, Magnesium-Aluminium-Silikat, Mono-und Polysaccharide, wie Dextran, Dextrin, Maltodextrin, Dextrat, Galactomannan, Glucose und direktkomprimierbare Zucker auf der Basis von Saccharose, Alginsäure und Alginate, wie Natriumalginat und Propylenglykolalginat, Zuckeralkohole, wie Sorbit, Mannit und Palatinit, Gelatine, Soja-Protein, Zein, Polyvinylpyrrolidon, Copovidon (Vinylpyrrolidon-Vinylacetat-Copolymer), Polymethacrylat, Polyacrylsäure, Polyacrylamid, Natriumstärkeglykolat, Polyethylenglykol 4000 bis 6000, Milchzucker, Rohrzucker und Calciumcarbonat.

4. Darreichungsform nach einem der voranstehenden Ansprüche, **dadurch gekennzeichnet, dass** das Trockenbindemittel ein Vinylpyrrolidon-Vinylacetat-Copolymer ist.

5. Darreichungsform nach einem der voranstehenden Ansprüche, **dadurch gekennzeichnet, dass** der Anteil der Darreichungsform an dem Trockenbindemittel 0,05 Gew.-% bis 20 Gew.-% beträgt, vorzugsweise 0,5 Gew.-% bis 10 Gew.-%.

6. Darreichungsform nach einem der voranstehenden Ansprüche, **dadurch gekennzeichnet, dass** der Anteil der Darreichungsform an Febuxostat 1 Gew.-% bis 40 Gew.-% beträgt, vorzugsweise 10 Gew.-% bis 30 Gew.-%.

7. Darreichungsform nach einem der voranstehenden Ansprüche, **dadurch gekennzeichnet, dass** das Febuxostat einen D10-Wert von 1 $\mu$m bis 5 $\mu$m, einen D50-Wert von 1 $\mu$m bis 15 $\mu$m und einen D90-Wert von 1,5 $\mu$m bis 20 $\mu$m aufweist.

8. Darreichungsform nach einem der voranstehenden Ansprüche, **dadurch gekennzeichnet, dass** die Darreichungsform mittels eines trockenen Verfahrens hergestellt ist.

9. Darreichungsform nach Anspruch 8, **dadurch gekennzeichnet, dass** die Darreichungsform mittels Direkttablettierung oder mittels Trockengranulierung hergestellt ist.

10. Darreichungsform nach einem der voranstehenden Ansprüche, **dadurch gekennzeichnet, dass** das Febuxostat die kristalline polymorphe Form A, M oder G aufweist.

11. Darreichungsform nach einem der voranstehenden Ansprüche, **dadurch gekennzeichnet, dass** die Darreichungsform eine innere granuläre Phase sowie eine äußere kontinuierliche Phase aufweist, wobei das Febuxostat in der inneren Phase und das Trockenbindemittel in der äußeren Phase enthalten ist.

12. Darreichungsform nach Anspruch 11, **dadurch gekennzeichnet, dass** das die innere Phase bildende Granulat einen D10-Wert von 1 $\mu$m bis 60 $\mu$m, einen D50-Wert von 20 $\mu$m bis 800 $\mu$m und einen D90-Wert von 100 $\mu$m bis 1600 $\mu$m aufweist.

13. Pulverförmige Mischung zur Herstellung einer Darreichungsform nach einem der Ansprüche 1 bis 12, **dadurch gekennzeichnet, dass** die Mischung Febuxostat sowie ein Trockenbindemittel umfasst, wobei das Trockenbindemittel einen D10-Wert von 1 $\mu$m bis 15 $\mu$m, einen D50-Wert von 10 $\mu$m bis 50 $\mu$m und einen D90-Wert von 30 $\mu$m bis 120 $\mu$m aufweist.

14. Verwendung eines Trockenbindemittels mit einem D10-Wert von 1 $\mu$m bis 15 $\mu$m, einem D50-Wert von 10 $\mu$m bis 50 $\mu$m und einem D90-Wert von 30 $\mu$m bis 120 $\mu$m bei der Herstellung einer Darreichungsform nach einem der Ansprüche 1 bis 12.

15. Verfahren zur Herstellung einer Darreichungsform nach einem der Ansprüche 1 bis 12, umfassend die Schritte

    - Bereitstellen einer pulverförmigen Mischung, enthaltend Febuxostat;
    - Trockengranulieren der Mischung unter Erhalt eines Trockengranulates;
    - Vermischen des Trockengranulats mit einem Trockenbindemittel und optional einem oder mehreren pharmazeutischen Hilfsstoffen, wobei das Trockenbindemittel einen D10-Wert von 1 $\mu$m bis 15 $\mu$m, einen D50-Wert von 10 $\mu$m bis 50 $\mu$m und einen D90-Wert von 30 $\mu$m bis 120 $\mu$m aufweist;
    - Verpressen der Mischung von Trockengranulat und Trockenbindemittel unter Erhalt einer Tablette und optional
    - Befilmen der Tablette.

Europäisches Patentamt
European Patent Office
Office européen des brevets

# EUROPÄISCHER RECHERCHENBERICHT

Nummer der Anmeldung
EP 15 00 2887

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | EP 2 902 016 A1 (ALFRED E TIEFENBACHER GMBH & CO KG [DE]) 5. August 2015 (2015-08-05) * Absätze [0001], [0011] - [0019]; Beispiele 1-8,11-13 * ----- | 1-15 | INV. A61K9/20 A61K31/426 |
| X | WO 2013/001441 A1 (RANBAXY LAB LTD [IN]; SINGLA AJAY KUMAR [IN]; GARG MUKESH [IN]; GUPTA) 3. Januar 2013 (2013-01-03) * Seite 2, Zeilen 15-30 * * Seite 3, Zeilen 1-3, 11-20 * * Seite 4, Zeilen 2,3 * * Seite 5, Absatz 16-21 * * Seite 7, Zeilen 1-9 * * Seite 10; Beispiel 2 * ----- | 1-15 | |

RECHERCHIERTE SACHGEBIETE (IPC)
A61K

Der vorliegende Recherchenbericht wurde für alle Patentansprüche erstellt

1

| Recherchenort | Abschlußdatum der Recherche | Prüfer |
|---|---|---|
| München | 4. Dezember 2015 | Toulacis, C |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 15 00 2887

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

04-12-2015

| Im Recherchenbericht angeführtes Patentdokument | Datum der Veröffentlichung | Mitglied(er) der Patentfamilie | Datum der Veröffentlichung |
|---|---|---|---|
| EP 2902016 A1 | 05-08-2015 | KEINE | |
| WO 2013001441 A1 | 03-01-2013 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2013001441 A
- EP 1020454 A
- CN 101085761


### In der Beschreibung aufgeführte Nicht-Patentliteratur


- Friabilität von nicht überzogenen Tabletten. Europäischen Arzneibuch. 411-412
- **A. RITSCHEL et al.** Die Tablette. Editio-Cantor-Verlag, 2002
- Bestimmung der Partikelgröße durch Laserdiffraktometrie. Ph. Eur. 455-460
- **A. BAUER-BRANDL et al.** Die Tablette. Editio-Cantor-Verlag, 2012